(19) Europäisches Patentamt
European Patent Office
Office européen des brevets

(11) Publication number : **0 417 036 B1**

# (12) EUROPEAN PATENT SPECIFICATION

(45) Date of publication of patent specification : **09.03.94 Bulletin 94/10**

(51) Int. Cl.$^5$ : **C07C 211/55,** C10M 133/12, // C10N30:10

(21) Application number : **90810636.2**

(22) Date of filing : **22.08.90**

## (54) Butenyl aromatic amine stabilizers.

The file contains technical information submitted after the application was filed and not included in this specification

(30) Priority : **30.08.89 US 400649**

(43) Date of publication of application : **13.03.91 Bulletin 91/11**

(45) Publication of the grant of the patent : **09.03.94 Bulletin 94/10**

(84) Designated Contracting States : **BE DE ES FR GB IT NL**

(56) References cited :
**DE-A- 2 005 843**
**FR-A- 2 103 486**
**GB-A- 1 438 482**
**US-A- 3 944 492**

(73) Proprietor : **CIBA-GEIGY AG**
**Klybeckstrasse 141**
**CH-4002 Basel (CH)**

(72) Inventor : **Babiarz, Joseph E.**
**P.O. Box 653, Pinesbridge Road**
**Amawalk, N.Y. 10501 (US)**
Inventor : **Rutsch, Werner, Dr.**
**Av. Weck-Reynold 1**
**CH-1700 Fribourg (CH)**

EP 0 417 036 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to 1,4-but-2-ene-bis-diarylamines compounds and to lubricant and mineral oil compositions which are stabilized against oxidative decomposition by the addition thereto of said 1,4-but-2-ene-bis-diarylamines.

### Background of the Invention

British Patent Specification 1 438 482 describes lubricant compositions containing aryl amines such as R-X, wherein R is *inter alia* a secondary amine residue of the following structure:

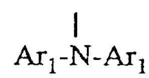

wherein the $Ar_1$ is an aromatic nucleus optionally bearing one or more substituents such as alkyl, alkenyl or alkoxy. The X group is a $C_3$- or $C_4$-alkenyl or alkynyl group attached to nitrogen wherein the unsaturation is in the β-position relative to the nitrogen atom. This specification also discloses compounds of the formula R-Y-R wherein R is as defined above and Y is a $C_4$-alkenyl or -alkynyl connecting group attached to the nitrogens wherein the unsaturation is β to each nitrogen. $Ar_1$ groups which are unsubstituted are not specifically taught in the patent.

### Summary of the Invention

It has now been discovered that certain tetraaryldinitrogen compounds show surprisingly high stabilizer activity and sufficient solubility in lubricant system. Thus, the subject matter of the instant invention relates to compounds of formula I and to lubricant compositions comprising a mineral oil, a synthetic oil or mixtures thereof and at least one compound of formula I.

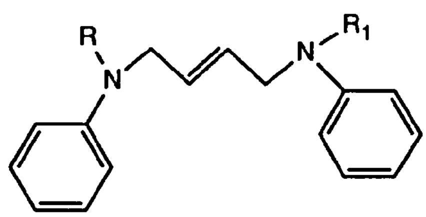

(I)

wherein R and $R_1$ are independently phenyl or 1-naphthyl.

Preferred are compounds of formula I as well as above-described compositions containing them, wherein R is phenyl and especially wherein $R_1$ and R are phenyl.

The invention further relates to a method of stabilizing lubricants against oxidative degradation comprising adding thereto at least one compound of formula I according to claim 1.

A compound of formula I as defined in claim 1 may be prepared by reacting a 1,4-dihalogeno-but-2-ene, preferably 1,4-dibromo-but-2-ene, with diphenyl amine or N-phenyl-naphth-1-yl amine or mixtures thereof.

The lubricants that come into consideration are known to the person skilled in the art and are described, for example, in Dieter Klamann "Schmierstoffe und verwandte Produkte", Verlag Chemie, Weinheim, 1982, in Schewe-Kobek,"Das Schmiermittel-Taschenbuch", Dr. Alfred Hüthig-Verlag, Heidelberg, 1974, or in "Ullmanns Encyclopädie der technischen Chemie", Vol. 13, pages 85-94 (Verlag Chemie, Weinheim, 1977).

Examples thereof are lubricants based on mineral oils, or synthetic lubricants especially those which are carboxylic acid ester derivatives and are used at temperatures of 200°C and above.

Examples of synthetic lubricants include lubricants based on a diester of a divalent acid with a monovalent alcohol, for example dioctyl sebacate or dinonyl adipate, a triester of trimethylol propane with a monovalent acid or with a mixture of such acids, for example trimethylolpropane tripelargonate, trimethylolpropane tricaprylate or mixtures thereof, a tetraester of pentaerythritol with a monovalent acid or with a mixture of such acids, for example pentaerythritol tetracaprylate, or a complex ester of monovalent and divalent acids with polyvalent

alcohols, for example a complex ester of trimethylolpropane with caprylic or sebacic acid or of a mixture thereof.

Especially suitable, in addition to mineral oils, are, for example, poly-α-olefins, lubricants based on esters, phosphates, glycols, polyglycols and polyalkylene glycols, and mixtures thereof with water.

Other lubricants suitable for compositions of the invention are natural oils, fats and waxes derived from plants and animals, or mixtures thereof. They may also be blended with the synthetic or mineral lubricants mentioned above. Examples are coconut-oil, olive-oil, rape-oil, linseed-oil, peanut-oil, soy-bean-oil, cotton-seed-oil, sunflower-seed-oil, pumpkin-seed-oil, corn-oil, fish-oil, beef tallow as well as modified, epoxidized or sulfoxidized forms thereof.

The compounds of formula I are readily soluble in lubricants and are therefore especially suitable as additives to lubricants and attention is drawn to their surprisingly good anti-oxidative action.

The compounds of formula I are able to display their superior properties, for example, in lubricants for internal combustion engines, for example internal combustion engines according to the Otto principle. For example, in lubricating oils the compounds of formula I prevent the formation of sludge or reduce sludge formation to a surprising extent.

It is also possible to prepare so-called master batches.

The compounds of formula I are effective even in very small amounts as additives in lubricants. They are advantageously mixed with the lubricants in an amount of from 0.01 to 5 % by weight, preferably in an amount of from 0.05 to 3 % by weight, and especially preferably in an amount of from 0.1 to 2 % by weight, in each case based on the lubricant.

The lubricants may, in addition, contain other additives which are added in order further to improve the basic properties of lubricants; these include: anti-oxidants, metal passivators, rust inhibitors, viscosity index enhancers, pour-point depressors, dispersants, detergents, high pressure additives and anti-wear additives.

For example, a number of such compounds can be found in the following list;

Examples of phenolic antioxidants

1. Alkylated Monophenols

2,6-Di-tert-butyl-4-methylphenol, 2,6-di-tert-butylphenol, 2-tert-butyl-4,6-dimethylphenol, 2,6-di-tert-butyl-4-ethylphenol, 2,6-di-tert-butyl-4-n-butylphenol, 2,6-di-tert-butyl-4-i-butylphenol, 2,6-di-cyclcopentyl-4-methylphenol, 2-(β-methylcyclohexyl)-4,6-dimethylphenol, 2,6-di-octadecyl-4-methylphenol, 2,4,6-tri-cyclohexylphenol, 2,6-di-tert-butyl-4-methoxymethylphenol, o-tert-butylphenol.

2. Alkylated Hydroquinones

2,6-Di-tert-butyl-4-methoxyphenol, 2,5-di-tert-butyl-hydroquinone, 2,5-di-tert-amylhydroquinone, 2,6-diphenyl-4-octadecyloxyphenol.

3. Hydroxylated Thiodiphenylethers

2,2'-Thio-bis-(6-tert-butyl-4-methylphenol), 2,2'-thio-bis-(4-octyl-phenol), 4,4'-thio-bis-(6-tert-butyl-3-methylphenol), 4,4'-thio-bis-(6-tert-butyl-2-methylphenol).

4. Alkylidene-Bisphenols

2,2'-Methylene-bis-(6-tert-butyl-4-methylphenol), 2,2'-methylene-bis-(6-tert-butyl-4-ethylphenol), 2,2'-methylene-bis-(4-methyl-6-(α-methylcyclo- hexyl)-phenol), 2,2'-methylene-bis-(4-methyl-6-cyclohexylphenol), 2,2'-methylene-bis-(6-nonyl-4-methylphenol), 2,2'-methylene-bis-(4,6-di-tert-butylphenol), 2,2'-ethylidene-bis-(4,6-di-tert-butylphenol), 2,2'-ethylidene-bis-(6-tert-butyl-4- or -5-isobutylphenol), 2,2'-methylene-bis-(6-(α-methylbenzyl-4-nonylphenol), 2,2'-methylene-bis-(6-(α,α-di-methylbenzyl)-4-nonylphenol), 4,4'-methylene-bis-(2,6-di-tert-butylphenol), 4,4'-methylene-bis-(6-tert-butyl-2-methylphenol), 1,1-bis-(5-tert-butyl-4-hydroxy-2-methylphenol)-butane, 2,6-di-(3-tert-butyl-5-methyl-2-hydroxybenzyl)-4-methylphenol, 1,1,3-tris-(5-tert-butyl-4-hydroxy-2-methylphenyl)-3-n-dodecyl)-mercaptobutane, ethyleneglycol-bis-[3,3-bis-(3'-tert-butyl-4'-hydroxyphenyl)-butyrate], bis-(3-tert-butyl-4-hydroxy-5-methylphenyl)-dicyclopentadiene, bis-[2-(3'-tert-butyl-2'-hydroxy-5'-methyl-benzyl)-6-tert-butyl-4-methyl-phenyl]-terephthalate.

5. Benzyl Compounds

1,3,5-Tri-(3,5-di-tert-butyl-4-hydroxybenzyl)-2,4,6-trimethyl-benzene, bis-(3,5-di-tert-butyl-4-hydroxybenzyl)-sulfide, 3,5-di-tert-butyl-4-hydroxybenzyl-mercaptoacetic acid-isooctylester, bis-(4-tert-butyl-3-hydroxy-2,6-dimethylbenzyl)dithiolterephthalate, 1,3,5-tris-(3,5-di-tert-butyl-4-hydroxybenzyl)-isocyanurate, 1,3,5-tris-(4-tert-butyl-3-hydroxy-2,6-dimethylbenzyl)-isocyanurate, 3,5-di-tert-butyl-4-hydroxybenzylphosphonic acid-dioctadecylester, 3,5-di-tert-butyl-4-hydroxybenzyl-phosphonic acid-monoethylester, calcium-salt.

6. Acylaminophenols

4-Hydroxy-lauric acid anilide, 4-hydroxy-stearic acid anilide, 2,4-bis-octylmercapto-6-(3,5-di-tert-butyl-4-hydroxyanilino)-s-triazine, N-(3,5-di-tert-butyl-4-hydroxyphenyl)-carbamic acid octyl ester.

7. Esters of β-(3,5-Di-tert-butyl-4-hydroxyphenol)-propionic acid

with mono- or polyhydric alcohols, for example with methanol, diethyleneglycol, octadecanol, triethyleneglycol, 1,6-hexanediol, pentaerythritol, neopentylglycol, tris-hydroxyethyl-isocyanurate, thiodiethyleneglycol, bis-hydroxyethyl-oxalic acid diamide.

8. Esters of β-(5-tert-butyl-4-hydroxy-3-methylphenyl)-propionic acid

with mono- or polyhydric alcohols, for example with methanol, diethyleneglycol, octadecanol, triethyleneglycol, 1,6-hexanediol, pentaerythritol, neopentylglycol, tris-hydroxyethyl-isocyanurate, thiodiethyleneglycol, di-hydroxyethyl-oxalic acid diamide.

9. Amides of β-(3,5-Di-tert-butyl-4-hydroxyphenyl)-propionic acid for example

N,N'-Bis-(3,5-di-tert-butyl-4-hydroxyphenylpropionyl)-hexamethylene-diamine, N,N'-bis-(3,5-di-tert-butyl-4-hydroxyphenylpropionyl)-tri-methylene-diamine, N,N'-bis-(3,5-di-tert-butyl-4-hydroxyphenylpropionyl)-hydrazine.

Examples of amine antioxidants:

N,N'-Di-isopropyl-p-phenylenediamine, N,N'-di-sec.-butyl-p-phenylene-diamine, N,N'-bis(1,4-dimethyl-pentyl)-p-phenylenediamine, N,N'-bis(1-ethyl-3-methylpentyl)-p-phenylenediamine, N,N'-bis(1-methyl-heptyl)-p-phenylenediamine, N,N'-cyclohexyl-p-phenylenediamine, N,N'-diphenyl-p-phenylenediamine, N,N'-di-(naphthyl-2-)-p-phenylenediamine, N-isopropyl-N'-phenyl-p-phenylenediamine, N-(1,3-dimethyl-butyl)-N'-phenyl-p-phenylenediamine, N-(1-methyl-heptyl)-N'-phenyl-p-phenylenediamine, N-cyclohexyl-N'-phenyl-p-phenylenediamine, 4-(p-toluenesulfonamido)-diphenylamine, N,N'-dimethyl-N,N'-di-sec-butyl-p-phenylenediamine, di-phenylamine, N-allyldiphenylamine, 4-isopropoxy-diphenylamine, N-phenyl-1-naphthylamine, N-phenyl-2-naphthylamine, octylated diphenylamine, e.g. p,p'-di-tert-octyldiphenylamine, 4-n-butylaminophenol, 4-butyrylamino-phenol, 4-nonanoylamino-phenol, 4-dodecanoylamino-phenol, 4-octadecanoyl-amino-phenol, di-(4-methoxy-phenyl)-amine, 2,6-di-tert-butyl-4-dimethyl-amino-methyl-phenol, 2,4'-diamino-diphenylmethane, 4,4'-diamino-diphenyl-methane, N,N,N',N'-tetramethyl-4,4'-diamino-diphenylmethane, 1,2-di- (phenylamino)-ethane, 1,2-di-[2-methyl-phenyl)-amino]-ethane, 1,3-di-(phenylamino)-propane, (o-tolyl)-biguanide, di-[4-(1',3'-dimethyl-butyl)-phenyl]amine, tert-octylated N-phenyl-1-naphthylamine, mixture of mono- and dialkylated tert-butyl-/tert-octyldiphenylamines, 2,3-dihydro-3,3-dimethyl-4H-1,4-benzothiazine, phenothiazine, n-allylphenothiazine.

Examples for other antioxidants:

Aliphatic or aromatic phosphites, esters of thiodipropionic acid or of thiodiacetic acid, or salts of dithiocarbamic or dithiophosphoric acid.

Examples of metal deactivators, for example for copper, are:

Triazoles, benzotriazoles and derivatives thereof, tolutriazole and derivatives thereof, 2-mercaptobenzothiazole, 2-mercaptobenzotriazole, 2,5-dimercaptothiadiazole, 2,5-dimercaptobenzotriazole, 5,5'-methylene-bis-benzotriazole, 4,5,6,7-tetrahydrobenzotriazole, salicylidene-propylene-diamine and salicylaminoguanidine and salts thereof.

Examples of rust inhibitors are:

a) Organic acids, their esters, metal salts and anhydrides, e.g. N-oleoyl-sarcosine, sorbitan-mono-oleate, lead-naphthenate, alkenyl-succinic acids and -anhydrides, e.g. dodecenyl-succinic acid anhydride, succinic acid partial esters and amides, 4-nonyl-phenoxy-acetic acid.
b) Nitrogen-containing compounds, e.g.
I. Primary, secondary or tertiary aliphatic or cycloaliphatic amines and amine-salts of organic and inorganic acids, e.g. oil-soluble alkylammonium carboxylates
II. Heterocyclic compounds, e.g.
substituted imidazolines and oxazolines.

c) Phosphorus-containing compounds, e.g.
Amine salts of phosphonic acid or phosphoric acid partial esters, zinc dialkyldithio phosphates.
d) Sulfur-containing compounds, e.g.
Barium-dinonylnaphthalene-n-sulfonates, calcium petroleum sulfonates.

Examples of viscosity-index improvers are:

Polyacrylates, polymethacrylates, vinylpyrrolidone/methacrylate-copolymers, polyvinylpyrrolidones, polybutenes, olefin-copolymers, styrene/acrylate-copolymers, polyethers.

Examples of pour-point depressants are:

Polymethacrylates, alkylated naphthalene derivatives.

Examples of dispersants/surfactants are:

Polybutenylsuccinic acid-amides or -imides, polybutenylphosphonic acid derivatives, basic magnesium-, calcium-, and bariumsulfonates and -phenolates.

Examples of anti-wear additives are:

Sulfur- and/or phosphorus- and/or halogen-containing compounds eg sulfurised vegetable oils, zinc dialkyldithiophosphates, tritolylphosphate, chlorinated paraffins, alkyl- and aryldi- and trisulfides, triphenylphos- phorothionate, diethanolaminomethyltolutriazole, di(2-ethylhexyl)-aminomethyltolutriazole.

The compounds of formula I can be prepared by substituting the halogen atoms, preferably bromine atoms, 1,4-dihalogeno (preferably dibromo)-but-2-ene with diphenylamine or N-phenyl-naphth-1-ylamino. The reaction is preferably carried out in the presence of an aqueous mixture of potassium iodide and a base, for example NaOH or KOH. Advantageously a phase transfer catalyst is also added.

The following examples will further illustrate the embodiments of the instant invention. Percentages and parts are by weight, unless otherwise stated.

Example 1: 1,4-Bis(diphenylamino)-2-butene

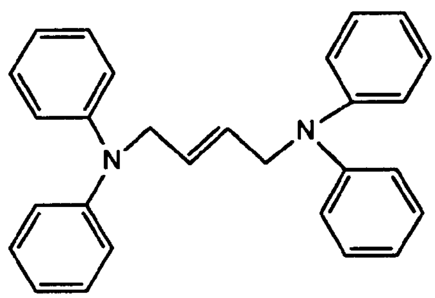

A vigorously stirred mixture of diphenylamine (150 g, 0.9 mol), 1,4-dibromo-but-2-ene (104 g, 0.49 mol), potassium iodide (8 g, 0.050 mol), tetrabutylammonium bromide (7 g, 0.025 mol), NaOH (43 g, 1.06 mol) and water (150 ml) is warmed to 60°C for five hours. The mixture is allowed to cool to room temperature, the solids are collected, washed with water and recrystallized from ethanol to give 69 grams (36 %) of a tan solid. M.P.: 105-107°C. Analysis: Calc'd. for $C_{28}H_{26}N_2$: C, 86.1; H, 6.7; N, 7.2; Found: C, 85.8; H, 6.5; N, 7.2.

Example 2: 1,4-Bis(N-phenyl-1-naphthylamino)-2-butene

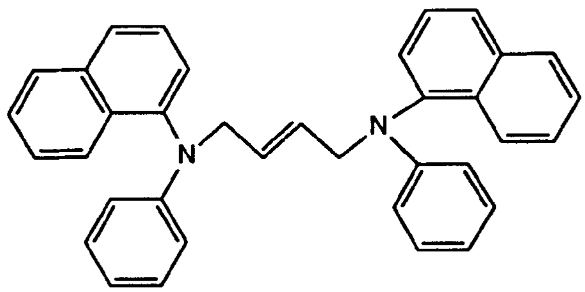

A mixture of N-phenyl-1-naphthylamine (63 g, 0.29 mol) and 1,4-dibromo-2-butene (12 g, 0.056 mol) is warmed to 70°C for 3 days. The resulting mass is allowed to cool and subsequently extracted with hot hexane (3 x 100 ml). The hexane extracts are concentrated in vacuo to an oil that is chromatographed ($SiO_2$, 95/5 cyclohexane/ether) giving an oil that is crystallized from ether yielding 4,8 g (17.5 %) of a tan solid. M.P.: 140-142°C.

Example 3: Engine Oil Thin Film Oxygen Uptake Test (TFOUT-Test)

The following example illustrates the antioxidant activity of the instant stabilizers.

The determination of stabilization effectiveness is carried out in the standard rotary bomb apparatus (ASTM D 2272) with modifications in procedure as described in the Reprint No. 82 CC-10-1, presented at the Conference of the American Society of Lubrication Engineers, October 5-7, 1982.

In the test apparatus, a 1.5 gram sample of 150 N paraffinic mineral oil (API 1119) is combined with enough zinc dialkyldithiophosphate (ZDTP) to give 0,1 % by weight of zinc and enough test compound to give 0.4 % or 0.5 % by weight, respectively, of the test compound. To this composition is added a catalyst package comprising 0.075 g of soluble metal catalyst mixture which is made by combining 0.69 parts of cupric naphthenate, 0.41 parts of ferric naphthenate, 8.0 parts of lead naphthenate, 0.36 parts of manganese naphthenate and 0.36 parts of stannous naphthenate.

The test results are presented in the following table with higher TFOUT values being indicative of increased antioxidant activity.

Table 1.

THIN FILM OXYGEN UPTAKE TEST (TFOUT)

| Compound Added to API 1119 | TFOUT Time in Minutes | |
|---|---|---|
| | 0.4 % | 0.5 % |
| 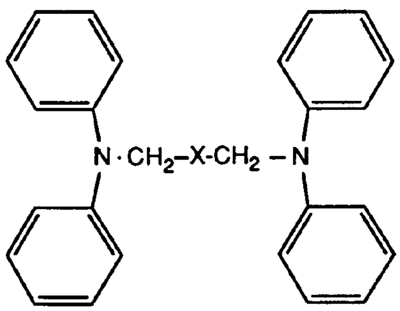 | | |
| X = CH=CH | 271 | 600+ |
| CONTROL - No Additive | 124 | |

It is clear from the data that small amounts of the instant stabilizers significantly improve the stability of the lubricant compositions as evidenced by the increased time needed to take up oxygen according to the TFOUT test.

## Claims

1. A compound of formula (I)

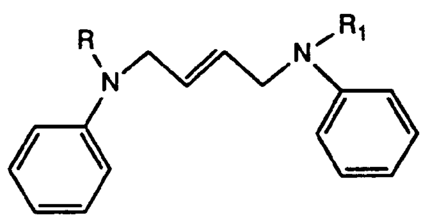

(I)

wherein R and $R_1$ are independently phenyl or 1-naphthyl.

2. A compound according to claim 1 wherein R is phenyl.

3. A compound according to claim 1 wherein R and $R_1$ are phenyl.

4. A lubricant composition comprising a mineral oil or a synthetic oil or mixtures thereof and at least one compound of formula (I)

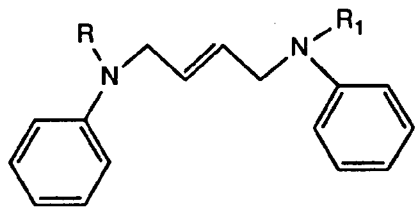

(I)

wherein R and $R_1$ are independently of one another phenyl or 1-naphthyl.

5. A lubricant composition according to claim 4 containing a compound of formula I wherein R is phenyl.

6. A lubricant composition according to claim 4 containing a compound of formula I wherein R and $R_1$ are phenyl.

7. A method of stabilizing lubricants against oxidative degradation comprising adding thereto at least one compound of formula I according to claims 1.

## Patentansprüche

1. Verbindung der Formel (I)

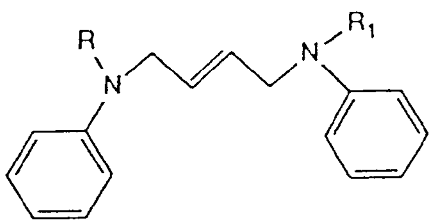

(I)

worin R und $R_1$ unabhängig voneinander Phenyl oder 1-Naphthyl sind.

2. Verbindung gemäß Anspruch 1, worin R Phenyl ist.

3. Verbindung gemäß Anspruch 1, worin R und $R_1$ Phenyl sind.

4. Schmiermittelzusammensetzung, umfassend ein Mineralöl oder ein synthetisches Öl oder Mischungen davon, und mindestens eine Verbindung der Formel (I)

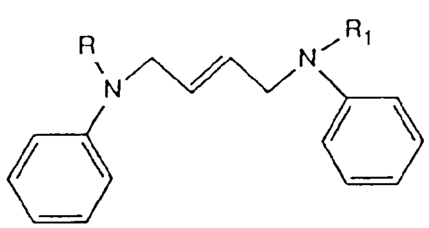

(I)

worin R und $R_1$ unabhängig voneinander Phenyl oder 1-Naphthyl sind.

5. Schmiermittelzusammensetzung gemäß Anspruch 4, enthaltend eine Verbindung der Formel (I), worin R Phenyl ist.

6. Schmiermittelzusammensetzung gemäß Anspruch 4, enthaltend eine Verbindung der Formel (I), worin

R und $R_1$ Phenyl sind.

7. Verfahren zum Stabilisieren von Schmiermitteln bzw. Schmierstoffen gegen oxidativen Abbau, umfassend die Zugabe von mindestens einer Verbindung der Formel (I) gemäß Anspruch 1 dazu.

**Revendications**

1. Composé de formule (I)

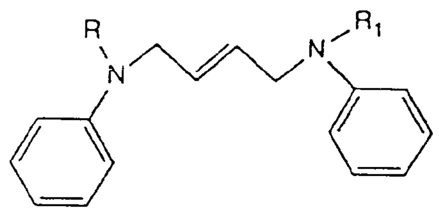

(I)

dans laquelle R et $R_1$ représentent chacun, indépendamment, un groupe phényle ou 1-naphtyle.

2. Composé selon la revendication 1, dans lequel R représente un groupe phényle.

3. Composé selon la revendication 1, dans lequel R et $R_1$ représentent chacun un groupe phényle.

4. Composition lubrifiante comprenant une huile minérale ou une huile synthétique ou un de leurs mélanges, et au moins un composé de formule (I)

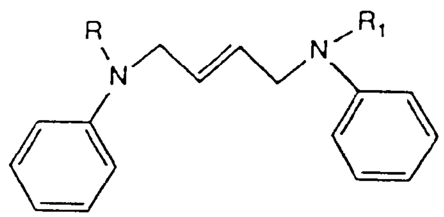

(I)

dans laquelle R et $R_1$ représentent chacun, indépendamment l'un de l'autre, un groupe phényle ou 1-naphtyle.

5. Composition lubrifiante selon la revendication 4, contenant un composé de formule I dans laquelle R représente un groupe phényle.

6. Composition lubrifiante selon la revendication 4, contenant un composé de formule I dans laquelle R et $R_1$ représentent chacun un groupe phényle.

7. Procédé pour stabiliser des lubrifiants à l'encontre d'une dégradation oxydante, ce procédé comprenant l'addition à ces lubrifiants d'au moins un composé de formule I selon la revendication 1.